# EUROPÄISCHE PATENTSCHRIFT

(11) **EP 2 538 899 B1**
(45) Veröffentlichungstag und Bekanntmachung des Hinweises auf die Patenterteilung: **18.06.2014**
(21) Anmeldenummer: 11703213.6
(22) Anmeldetag: 08.02.2011
(51) Int. Cl.: A61G 5/10, A61F 5/37

(54) **POSITIONIERUNGSVORRICHTUNG UND VERFAHREN ZUM POSITIONIEREN VON KÖRPERBEHINDERTEN PERSONEN IN EINE SITZSTELLUNG**
POSITIONING DEVICE AND METHOD FOR POSITIONING PHYSICALLY HANDICAPPED PERSONS IN A SEATED POSITION
DISPOSITIF DE POSITIONNEMENT ET PROCÉDÉ POUR POSITIONNER DES PERSONNES HANDICAPÉES DANS UNE POSITION ASSISE

(30) Priorität: 26.02.2010 DE 102010009537
(43) Veröffentlichungstag der Anmeldung: 02.01.2013
(73) Patentinhaber: Wettengel, René, 47445 Moers (DE); Knoche, Volker, 47051 Duisburg (DE)
(72) Erfinder: Wettengel, René, 47445 Moers (DE); Knoche, Volker, 47051 Duisburg (DE)
(74) Vertreter: Von Kreisler Selting Werner - Partnerschaft von Patentanwälten und Rechtsanwälten mbB
(86) Internationale Anmeldenummer: PCT/EP2011/051818
(87) Internationale Veröffentlichungsnummer: WO 2011/104113

(56) Entgegenhaltungen:
- DE-A1- 10 304 785
- US-B1- 7 097 628

## Beschreibung

Die vorliegende Erfindung betrifft eine Positionierungsvorrichtung und ein Verfahren zum Positionieren von körperbehinderten Personen in eine Sitzstellung.

Bei körperbehinderten Personen mit Muskelerkrankungen oder spastischen Lähmungen besteht häufig das Problem, dass aufgrund von Muskelversteifungen oder Muskelverkrampfungen ein Positionieren der Personen in eine Sitzstellung nur mit sehr großem Aufwand möglich ist. Zumeist werden die Personen von Betreuern oder Pflegern unter großem Kraftaufwand in die entsprechende Sitzstellung gedrückt.

Diese Prozedur ist sowohl für die körperbehinderte Person als auch für den Pfleger, bzw. Betreuer unbequem, zumal die körperbehinderte Person häufig lange in einer Stellung gehalten werden muss, bis sich die Muskeln entkrampfen bzw. sich entspannen.

In einer Sitzposition haben derartige körperbehinderte Personen häufig eine Fehlhaltung, die zu Wirbelsäulenschäden führen kann. Aus diesem Grund schlägt die DE 10304785 A1 einen körperangepassten, offenen Stützring aus elastischem Material vor, der seitliche Anlegezungen sowie eine sich gegen das Schambein abstützende Schambeinanlage aufweist. Der Stützring ist mit mehreren Befestigungsmitteln an der Sitzlehne und der Sitzfläche eines Sitzes, beispielsweise an einem Rollstuhl, befestigt. Die vorgeschlagene Vorrichtung ermöglicht lediglich eine stützende Wirkung in der Sitzposition, um die Wirbelsäulenfehlhaltung zu vermeiden.

Die körperbehinderte Person muss jedoch weiterhin von den Betreuern oder Pflegern in die entsprechende Sitzposition gedrückt werden. Lediglich bei einem Ausführungsbeispiel, bei dem ein verschiebbares Sitzuntergestell vorgesehen ist, kann die Positionierung der körperbehinderten Person mit Hilfe des Sitzuntergestells im geringen Maße unterstützt werden. Aufgrund der offenen und elastischen Ausgestaltung des Stützrings ist eine Positionierung der körperbehinderten Person über den Stützring nicht möglich, da bei dem Aufbringen von Kräften auf den Stützring dieser sich aufbiegen würde, sodass die Person aus dem Stützring herausrutschen kann. Darüber hinaus besteht die Gefahr, dass beim Positionieren der körperbehinderten Person über die vorgeschlagene Vorrichtung Verletzungen durch die Anlegezungen oder die Schambeinanlage entstehen können. Aufgrund der Schambeinanlage ist darüber hinaus eine vorteilhafte Sitzposition nicht oder nur eingeschränkt möglich.

Um die sowohl für Betreuer, bzw. Pfleger als auch die körperbehinderte Person unbequeme Positionierungsprozedur zu vereinfachen, wäre daher eine Vorrichtung zur Positionierung der körperbehinderten Personen wünschenswert.

Es ist daher die Aufgabe der vorliegenden Erfindung, eine Positionierungsvorrichtung sowie ein Verfahren zum Positionieren einer körperbehinderten Person in eine Sitzstellung bereitzustellen, die das Positionieren der körperbehinderten Person auf eine einfache Art und Weise ermöglicht ohne dass ein Betreuer oder Pfleger die körperbehinderte Person in aufwändiger Art und Weise in die Sitzposition drücken muss.

Zur Lösung der Aufgabe, dienen die Merkmale des Anspruchs 1 sowie das Verfahren nach Anspruch 12.

Erfindungsgemäß ist vorgesehen, dass eine Positionierungsvorrichtung zum Positionieren einer körperbehinderten Person in eine Sitzstellung, vorzugsweise in einem Rollstuhl, einen Sitz mit einer Basis und einer gegenüber der Basis verschiebbaren Sitzfläche aufweist. Ferner ist eine formstabile Positionierungsmanschette vorgesehen, die am Beckenbereich der Person formschlüssig anlegbar ist und sich im angelegten Zustand mit Auflageflächen auf dem Beckenkamm der Person abstützt. An der Positionierungsmanschette sind Zugmittel vorgesehen, die die Positionierungsmanschette mit der Basis des Sitzes verbinden, wobei die Zugmittel jeweils in einem Seitenbereich der Positionierungsmanschette befestigt sind und eine Betätigungseinrichtung aufweisen. Bei Betätigung der Zugmittel können die Zugmittel auf die Positionierungsmanschette eine Zugkraft in Richtung der Sitzfläche und in einer Richtung entgegen der Sitzrichtung ausüben, wodurch die Person in die Sitzstellung ziehbar ist.

Die erfindungsgemäße Positionierungsvorrichtung hat den Vorteil, dass mit Hilfe der Positionierungsmanschette die körperbehinderte Person in die Sitzstellung ziehbar ist. Dabei ist es nicht notwendig, dass ein Pfleger oder Betreuer die körperbehinderte Person in aufwändiger Art und Weise drückt. Durch das Vorsehen einer formstabilen Positionierungsmanschette wird vermieden, dass während des Positionierungsvorgangs die körperbehinderte Person aus der Manschette herausrutschen kann. Darüber hinaus ermöglicht die erfindungsgemäße Positionierungsvorrichtung, dass die körperbehinderte Person zunächst in eine Stellung, die annähernd der Sitzstellungen ist, positioniert werden kann und mit Hilfe der Positionierungsmanschette in dieser Stellung gehalten werden kann. In dieser Stellung können sich die Muskeln der körperbehinderten Person entspannen bzw. entkrampfen, sodass danach ein Verbringen der körperbehinderten Person in die endgültige Sitzstellung möglich ist. Durch das Vorsehen einer gegenüber einer Basis verschiebbaren Sitzfläche und das Verbinden der Positionierungsmanschette mit der Basis der Sitzfläche, kann mit Hilfe der Zugmittel die verschiebbare Sitzfläche beim Positionieren der körperbehinderten Personen verschoben werden, sodass die verschiebbare Sitzfläche den Positionierungsvorgang unterstützt und darüber hinaus einen, für die körperbehinderte Person bequemeren Positionierungsvorgang ermöglicht.

Die erfindungsgemäße Positionierungsvorrichtung kann sowohl mit einem Rollstuhl als auch mit einem herkömmlichen Stuhl verwendet werden oder Teil von diesem sein. Mit Hilfe der Zugmittel kann die Person zu der Sitzfläche und in Richtung einer Sitzlehne gezogen werden. Unter Basis des Sitzes im Rahmen der Erfindung wird ein gegenüber der Sitzfläche ortsfester Teil des Sitzes verstanden. Bei einem Ausführungsbeispiel, bei dem der Sitz Teil eines Stuhl oder Rollstuhls ist, kann somit die Basis auch ein Teil des Stuhls oder Rollstuhls sein.

Vorzugsweise ist vorgesehen, dass die Positionierungsmanschette geschlossen ist, wobei die Positionierungsmanschette beispielsweise einen Verschluss aufweisen kann. Durch die geschlossene Positionierungsmanschette kann beispielsweise die Formstabilität der Positionierungsmanschette gewährleistet werden, selbst dann, wenn die Positionierungsmanschette aus einem eher elastischen Material hergestellt ist. Darüber hinaus ermöglicht der Verschluss, dass die Positionierungsmanschette in vorteilhafter Weise am Beckenbereich der Person formschlüssig anlegbar ist.

In einem Ausführungsbeispiel der Erfindung ist vorgesehen, dass zwei Zugmittel vorgesehen sind, wobei jeweils ein Zugmittel an einer Seite der Positionierungsmanschette angeordnet ist. Durch das Vorsehen von jeweils einem Zugmittel an einer Seite der Positionierungsmanschette kann die körperbehinderte Person gleichmäßig in die Sitzstellung gezogen werden, wobei gleichzeitig gewährleistet wird, dass die Zugmittel gut zugänglich sind und somit auf einfache Art und Weise betätigbar sind.

Es kann vorgesehen sein, dass die Positionierungsmanschette mehrteilig ist, wobei die mehreren Teile der Positionierungsmanschette durch Steck- oder Gelenkverbindungen miteinander verbindbar, bzw. verbunden sind. Durch das Vorsehen einer derartigen Positionierungsmanschette ist die Bereitstellung einer besonders formstabilen Positionierungsmanschette auf einfache Art und Weise möglich, wobei gleichzeitig gewährleistet wird, dass die Positionierungsmanschette auf einfache Art und Weise am Beckenbereich der Person formschlüssig anlegbar ist.

Dabei kann vorgesehen sein, dass die Positionierungsmanschette aus mehreren Rohrteilen besteht. Dadurch ist es möglich, die Positionierungsmanschette auf eine einfachen Art und Weise und kostengünstig herzustellen.

Nach einem Ausführungsbeispiel ist vorgesehen, dass die Positionierungsvorrichtung eine Rückenanlage aufweist. Durch die Rückenanlage wird ermöglicht, dass die Positionierungsmanschette im angelegten Zustand bequem für die Person tragbar ist, wobei gleichzeitig verhindert werden kann, dass sich die Positionierungsmanschette beispielsweise verdrehen oder verkannten kann.

Die Zugmittel können Spannbänder, Spannriemen, Spanngurte oder Zugstangen sein.

Nach einem Ausführungsbeispiel der Erfindung kann vorgesehen sein, dass die Sitzfläche an der Basis arretierbar ist, vorzugsweise in zwei Positionen. Dadurch wird ermöglicht, dass die körperbehinderte Person beispielsweise zunächst in eine erste Körperstellung gezogen wird, in der die Sitzfläche arretiert wird. Nach einem Entspannen bzw. Entkrampfen der Muskulatur kann die Person danach in die endgültige Sitzstellung gezogen werden, in der die Sitzfläche dann wiederum arretiert wird.

In einer vorteilhaften Ausgestaltung der Erfindung kann vorgesehen sein, dass der Sitz einen Motor aufweist, über den die Sitzfläche zum Verschieben antreibbar ist, wobei der Motor vorzugsweise ein Elektromotor ist. Durch das Vorsehen eines Motors kann das Positionieren der körperbehinderten Person unterstützt werden, sodass über die Zugmittel noch eine geringere Zugkraft ausgeübt werden muss. Die Verwendung eines Elektromotors ist dabei besonders vorteilhaft, da beispielsweise der Elektromotor über eine Batterie versorgt werden kann. Bei der Verwendung der erfindungsgemäßen Positionierungsvorrichtung in einem elektrischen Rollstuhl kann beispielsweise der Elektromotor des Sitzes über die Rollstuhlbatterie versorgt werden.

Die Erfindung sieht ferner die Verwendung einer erfindungsgemäßen Positionierungsvorrichtung zum Positionieren von körperbehinderten Personen in eine Sitzstellung, vorzugsweise in einem Rollstuhl vor. Dabei kann vorgesehen sein, dass in der Sitzstellung der Körper der körperbehinderten Person zwischen Rumpf und Oberschenkel ein Winkel zwischen 80 ° und 100 °, vorzugsweise zwischen 85 ° und 90 ° aufweist.

Die Erfindung sieht ferner ein Verfahren zum Positionieren von körperbehinderten Personen in eine Sitzstellung, vorzugsweise in einem Rollstuhl vor mit folgenden Schritten:
- formschlüssiges Anlegen einer formstabilen Positionierungsmanschette am Beckenbereich der Person, derart, dass sich die Positionierungsmanschette mit Auflageflächen auf dem Beckenkamm der Person abstützt,
- Setzen der Person auf eine verschiebbare Sitzfläche,
- Ziehen der Positionierungsmanschette in Richtung der Sitzfläche und in eine Richtung entgegen der Sitzrichtung und gleichzeitiges Verschieben der Sitzfläche in Richtung entgegen der Sitzrichtung derart, dass die Person in die Sitzstellung ziehbar ist.

Dabei kann vorgesehen sein, dass in der Sitzstellung der Körper der Person zwischen Rumpf und Oberschenkel einen Winkel zwischen 80 ° und 100 °, vorzugsweise zwischen 85 ° und 90 ° aufweist.

Bei dem erfindungsgemäßen Verfahren kann eine erfindungsgemäße Positionierungsvorrichtung vorgesehen sein.

Im Folgenden wird unter Bezugnahme auf die nachfolgenden Figuren die Erfindung näher erläutert. Es zeigen:
- Fig. 1: eine schematische Seitenansicht einer erfindungsgemäßen Positionierungsvorrichtung in einem Rollstuhl
- Fig. 2: eine schematische Draufsicht der Positionierungsmanschette einer erfindungsgemäßen Positionierungsvorrichtung sowie
- Fign. 3 u. 4: Ansichten eines zweiten Ausführungsbeispiels einer Positionierungsmanschette einer erfindungsgemäßen Positionierungsvorrichtung.

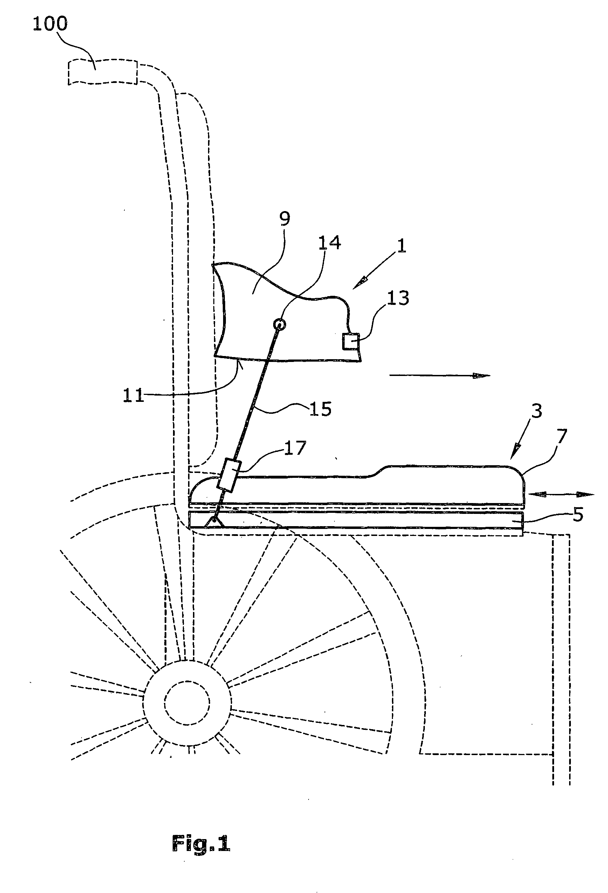

In Fig.1 ist ein erstes Ausführungsbeispiel einer erfindungsgemäßen Positionierungsvorrichtung 1 zum Positionieren einer körperbehinderten Person in einer Sitzstellung schematisch in einer Seitenansicht dargestellt. Die Positionierungsvorrichtung 1 ist auf eine Sitzfläche eines Rollstuhls 100 aufgesetzt. Der Einfachheit halber ist in Fig. 1 die körperbehinderte Person nicht dargestellt.

Die Positionierungsvorrichtung 1 besteht aus einem Sitz 3 mit einer Basis 5 und einer gegenüber der Basis 5 verschiebbaren Sitzfläche 7. Die Basis 5 kann beispielsweise an der Sitzfläche des Rollstuhls befestigt werden.

Die Sitzfläche 7 ist beispielsweise mit Hilfe von in Fig. 1 nicht dargestellten Schienen an die Basis 5 geführt und ist in Sitzrichtung bzw. entgegen der Sitzrichtung verschiebbar. Die Sitzrichtung ist in Fig. 1 durch einen Pfeil angedeutet.

Die Positionierungsvorrichtung 1 weist ferner eine formstabile Positionierungsmanschette 9 auf, die derart geformt ist, dass die Positionierungsmanschette 9 am Beckenbereich einer körperbehinderten Person formschlüssig anlegbar ist. Im unteren Bereich weist die Positionierungsmanschette 9 Auflageflächen 11 auf, mit der sich die Positionierungsmanschette 11 im angelegten Zustand auf dem Beckenkamm der Person abstützt.

Im vorderen Bereich weist die Positionierungsmanschette 9 einen Verschluss 13 auf. Auf diese Weise kann gewährleistet sein, dass die Positionierungsmanschette 9 aus einem elastischen Material gefertigt sein kann, wobei durch den Verschluss die Formstabilität der Positionierungsmanschette hergestellt werden kann. Die Positionierungsmanschette 9 kann somit in vorteilhafter Weise durch elastische Verbiegung an dem Beckenbereich der Person angelegt werden und anschließend durch Verschließen des Verschlusses in einen formstabilen Zustand gebracht werden.

In einem Seitenbereich der Positionierungsmanschette 9 sind Zugmittel 15 angeordnet, die die Positionierungsmanschette 9 mit der Basis 5 des Sitzes 3 verbinden. Die Zugmittel 15 sind über Anlenkpunkte 14 mit der Positionierungsmanschette 9 verbunden und weisen jeweils Betätigungseinrichtungen 17 auf, über die mit Hilfe der Zugmittel eine Zugkraft auf die Positionierungsmanschette ausgeübt werden kann. Dadurch dass die Zugmittel seitlich an der Positionierungsmanschette sowie am, entgegen der Sitzrichtung, äußeren Ende der Basis 5 befestigt sind, können die Zugmittel 15 auf die Positionierungsmanschette eine Kraft in Richtung der Sitzfläche 7 und in eine Richtung entgegen der Sitzrichtung ausüben. Dadurch kann eine körperbehinderte Person, an der die Positionierungsmanschette angelegt ist, mit Hilfe der Zugmittel in eine Sitzstellung gezogen werden, in dem die Zugkraft über die Positionierungsmanschette 9 auf den Beckenkamm der Person und somit auf die Person übertragen wird, sodass die Person in die Sitzstellung gedrückt wird.

Dabei kann die Sitzfläche 7 zunächst gegenüber der Basis 5 in Sitzrichtung verschoben sein, sodass die Sitzfläche 7 bei dem Positionieren der körperbehinderten Person durch die Zugkraft in die Richtung entgegen der Sitzrichtung und somit in Richtung der Sitzlehne des Rollstuhls gezogen wird. Dadurch wird das Positionieren der körperbehinderten Person in die Sitzstellung unterstützt.

Die formschlüssige Anlage der Positionierungsmanschette 9 am Beckenbereich der Person sowie die formstabile Ausgestaltung der Positionierungsmanschette 9 ermöglichen, dass die Zugkraft auf den Beckenkamm der Person übertragen und ein Verdrehen des Beckens verhindert wird.

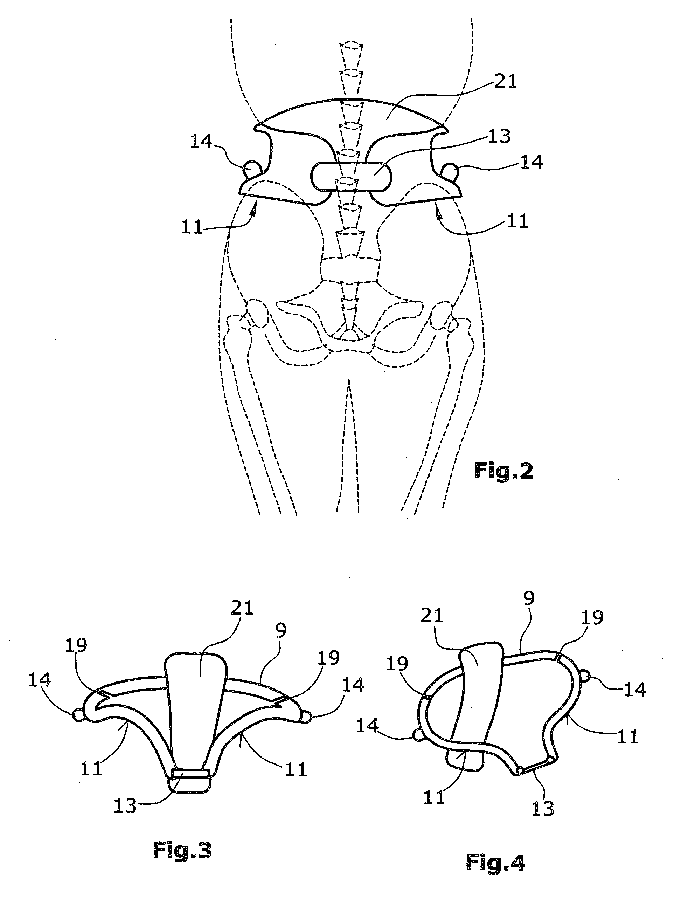

In Fig. 2 ist ein Ausführungsbeispiel einer Positionierungsmanschette 9 für eine erfindungsgemäße Positionierungsvorrichtung schematisch im an den Körper einer körperbehinderten Person angelegten Zustand gezeigt.

Die Positionierungsmanschette 9 weist eine Rückenanlage 21 auf, die im angelegten Zustand an dem Rücken der Person anliegt. Über einen Verschluss 13 ist die Positionierungsmanschette 9 im vorderen Bereich verschlossen, sodass eine Formstabilität der Positionierungsmanschette 9 gegeben ist.

Im unteren Bereich weist die Positionierungsmanschette 9 Auflageflächen 11 auf, die sich auf dem Beckenkamm der Person abstützen. Im seitlichen Bereich der Positionierungsmanschette sind zwei anliegende Punkte 14 angeordnet, an die in Fig. 2 nicht dargestellte Zugmittel 2 befestigt werden können. Dadurch kann auf die Positionierungsmanschette 9 eine Zugkraft ausgeübt werden, die die Person in die Sitzstellung drückt.

In Fig. 3 und 4 ist ein zweites Ausführungsbeispiel einer Positionierungsmanschette 9 einer erfindungsgemäßen Positionierungsvorrichtung gezeigt. In dem in Fig. 4 dargestellten Ausführungsbeispiel ist die Positionierungsmanschette weitestgehend aus einer Rohrkonstruktion gebildet, wobei die Rohrkonstruktion eine Rückanlage 21 aufweist. Die Rohrkonstruktion ist derart gebogen, dass die Positionierungsmanschette 9 an dem Körper der körperbehinderten Person angepasst ist. Im vorderen Bereich weist die Positionierungsmanschette einen Verschluss 13 auf. Da die Rohrkonstruktion eine geringe Flexibilität aufweist, sind Gelenkverbindungen 19 angeordnet, sodass nach Öffnen des Verschlusses 13 die Rohrkonstruktion teilweise aufgeschwenkt werden kann, sodass ein An- und Ablegen der Positionierungsmanschette 9 auf einfache Art und Weise möglich ist. Im Seitenbereich der Positionierungsmanschette 9 sind Anlenkpunkte 14 angeordnet, an die Zugmittel, die in Fig. 3 und 4 nicht dargestellt sind, angelenkt werden können.

Die Rohrkonstruktion weist im unteren Bereich Auflageflächen 11 auf, die sich im angelegten Zustand auf dem Beckenkamm der körperbehinderten Person abstützen.

Bei einer erfindungsgemäßen Positionierungsvorrichtung können die Zugmittel beispielsweise Spannbänder, Spannriemen, Spanngurte oder Zugstangen sein. Die Zugstangen können Beispielsweise als Gewindestangen ausgebildet sein, wobei als Betätigungsvorrichtung eine Schraubmuffe mit gegenläufigen Gewinde dienen kann. Bei Spannbändern oder Spanngurten kann als Betätigungsvorrichtung ein Gurtspanner dienen.

Der Sitz kann einen Motor aufweisen, mit dem die Sitzfläche zum Verschieben antreibbar ist. Der Motor kann beispielsweise als Elektromotor ausgestaltet sein. Auf diese Weise ist die Positionierung der körperbehinderten Person auf besonders einfache Art und Weise möglich, da der Motor durch das Verschieben der Sitzfläche das Positionieren der körperbehinderten Person unterstützt. Der Motor des Sitzes kann auch beispielsweise die Betätigungsvorrichtung der Zugmittel antreiben, so dass diese automatisch betätigt werden. Selbstverständlich kann auch ein separater Antrieb für die Betätigungsvorrichtungen der Zugmittel vorgesehen sein. Die Betätigungsvorrichtungen der Zugmittel können auch durch einen mechanischen Antrieb, der mit der Sitzfläche des Sitzes gekoppelt ist und durch das Verschieben der Sitzfläche betätigt wird, gebildet sein oder angetrieben werden. Beispielsweise kann die Betätigungsvorrichtung durch eine Spule gebildet sein, die durch das Verschieben der Sitzfläche gedreht wird und das Zugmittel aufrollt. An der Basis kann dann das Zugmittel beispielsweise um eine Umlenkrolle laufen.

Eine körperbehinderte Person kann nun folgendermaßen mit der erfindungsgemäßen Positionierungsvorrichtung positioniert werden. Zunächst wird die Positionierungsmanschette im Beckenbereich der körperbehinderten Person angelegt, sodass die Auflageflächen sich an dem Beckenkamm der körperbehinderten Person abstützen und die Rückenanlage am Rücken der Person anliegt. Der Verschluss der Positionierungsmanschette wird nun verschlossen, sodass eine Formstabilität der Positionierungsmanschette gegeben ist. Durch die entsprechende Anpassung der Positionierungsmanschette an den Körper der körperbehinderten Person ist nunmehr ein Verdrehen der Positionierungsmanschette nicht mehr möglich.

Die körperbehinderte Person wird nun auf die in Sitzrichtung verschobene Sitzfläche des Sitzes gesetzt. Zwischen den Anlenkpunkten einer Positionierungsmanschette und der Basis des Sitzes werden nun Zugmittel angebracht. Mit Hilfe der Betätigungsvorrichtung der Zugmittel wird eine Zugkraft auf die Positionierungsmanschette in Richtung der Sitzfläche und in eine Richtung entgegen der Sitzrichtung aufgebracht. Dadurch wird die körperbehinderte Person in die Sitzstellung gezogen, wobei gleichzeitig die Sitzfläche entgegen der Sitzrichtung gezogen wird. Dabei kann vorgesehen sein, dass die Sitzfläche in einer ersten Position einrastet, in der die Sitzstellung noch nicht ganz erreicht ist. Dies hat den Vorteil, dass bei Spastikern, die eine starke Verkrampfung der Beckenmuskulatur haben, die Person zunächst in dieser Vorstellung positioniert werden kann, bis sich die Muskulatur entsprechend entspannt bzw. entkrampft hat. Danach kann die Person in die endgültige Sitzstellung gezogen werden. Idealerweise besitzt der Körper der körperbehinderten Person in der Sitzstellung zwischen Rumpf und Oberschenkel einen Winkel zwischen 80 ° und 100 °, vorzugsweise < 90 °.

Es hat sich herausgestellt, dass das Positionieren einer körperbehinderten Person, insbesondere eines Spastikers, in eine Sitzstellung, bei der der Körper der Person zwischen Rumpf und Oberschenkel einen Winkel zwischen 85 °und 90 ° aufweist, das Verhalten der Person in positiver Weise beeinflusst wird.

Die erfindungsgemäße Positionierungsvorrichtung 1 stabilisiert dabei lediglich das Becken der körperbehinderten Person. Es hat sich herausgestellt, dass insbesondere bei Spastikern, die häufig unter einer Beckenversteifung leiden, die Positionierung in der Sitzstellung sowie die Stabilisierung des Beckens zu einer verbesserten Rumpfkontrolle sowie einer besseren Kontrolle des gesamten Bewegungsapparates führen. Überraschender Weise verbessert sich bei einer Positionierung eines Spastikers mit der erfindungsgemäßen Positionierungsvorrichtung bereits nach kurzer Zeit das Schluckverhalten des Spastikers, sodass eine Verminderung des Speichelausflusses erreicht werden kann. Auch hat sich herausgestellt, dass mit Hilfe der Positionierungsvorrichtung positionierte, spastisch behinderte Personen eine geistig bessere Aufnahme besitzen.

## Patentansprüche

1. Positionierungsvorrichtung (1) zum Positionieren einer körperbehinderten Person in eine Sitzstellung, vorzugsweise in einem Rollstuhl,
- mit einem Sitz (3) mit einer Basis (5) und mit einer gegenüber der Basis (5) verschiebbaren Sitzfläche (7),
- mit einer formstabilen Positionierungsmanschette (9), wobei die Positionierungsmanschette (9) am Beckenbereich der Person formschlüssig anlegbar ist und sich im angelegten Zustand mit Auflageflächen (11) auf dem Beckenkamm der Person abstützt,
- mit Zugmitteln (15), die der Positionierungsmanschette (9) mit der Basis (5) des Sitzes (3) verbinden, wobei die Zugmittel (15) jeweils an einem Seitenbereich der Positionierungsmanschette (9) befestigt sind und eine Betätigungseinrichtung (17) aufweisen,
- wobei die Zugmittel (15) bei Betätigung auf die Positionierungsmanschette (9) eine Zugkraft in Richtung der Sitzfläche (7) und in eine Richtung entgegen der Sitzrichtung ausüben, wodurch die Person in die Sitzstellung ziehbar ist.

2. Positionierungsvorrichtung nach Anspruch 1, **dadurch gekennzeichnet, dass** die Positionierungsmanschette (9) geschlossen ist, wobei die Positionierungsmanschette (9) vorzugsweise einen Verschluss (13) aufweist.

3. Positionierungsvorrichtung nach Anspruch 1 oder 2, **dadurch gekennzeichnet, dass** zwei Zugmittel (15) vorgesehen sind, wobei jeweils ein Zugmittel (15) an einer Seite der Positionierungsmanschette (9) angeordnet ist.

4. Positionierungsvorrichtung nach einem der Ansprüche 1 bis 3, **dadurch gekennzeichnet, dass** die Positionierungsmanschette (9) mehrteilig ist, wobei die mehreren Teile der Positionierungsmanschette (9) durch Steck- oder Gelenkverbindungen (19) miteinander verbindbar bzw. verbunden sind.

5. Positionierungsvorrichtung nach Anspruch 4, **dadurch gekennzeichnet, dass** die Positionierungsmanschette (9) aus mehreren Rohrteilen besteht.

6. Positionierungsvorrichtung nach einem der Ansprüche 1 bis 5, **dadurch gekennzeichnet, dass** die Positionierungsvorrichtung (9) eine Rückenanlage (21) aufweist.

7. Positionierungsvorrichtung nach einem der Ansprüche 1 bis 6, **dadurch gekennzeichnet, dass** die Zugmittel (15) Spannbänder, Spannriemen, Spanngurte oder Zugstangen sind.

8. Positionierungsvorrichtung nach einem der Ansprüche 1 bis 7, **dadurch gekennzeichnet, dass** die Sitzfläche (7) an der Basis (5) arretierbar ist, vorzugsweise in mindestens zwei Positionen.

9. Positionierungsvorrichtung nach einem der Ansprüche 1 bis 8, **dadurch gekennzeichnet, dass** der Sitz einen Motor aufweist, über den die Sitzfläche (7) zum Verschieben antreibbar ist, wobei der Motor vorzugsweise ein Elektromotor ist.

10. Verwendung einer Positionierungsvorrichtung (1) nach einem der Ansprüche 1 bis 9 zum Positionieren von körperbehinderten Personen in eine Sitzstellung, vorzugsweise in einem Rollstuhl (10).

11. Verwendung nach Anspruch 10, **dadurch gekennzeichnet, dass** in der Sitzstellung der Körper der Person zwischen Rumpf und Oberschenkeln einen Winkel zwischen 80° und 100°, vorzugsweise zwischen 85° und 90°, aufweist.

12. Verfahren zur Positionierung von körperbehinderten Personen in eine Sitzstellung, vorzugsweise in einem Rollstuhl, **gekennzeichnet durch** folgende Schritte:
- formschlüssiges Anlegen einer formstabilen Positionierungsmanschette (9) am Beckenbereich der Person, derart, dass sich die Positionierungsmanschette (9) mit Auflageflächen (11) auf dem Beckenkamm der Person abstützt
- Setzen der Person auf eine verschiebbare Sitzfläche (7)
- Ziehen der Positionierungsmanschette (9) in Richtung der Sitzfläche (7) und in eine Richtung entgegen der Sitzrichtung und gleichzeitiges Verschieben der Sitzfläche (7) in Richtung entgegen der Sitzrichtung, derart, dass die Person in die Sitzstellung ziehbar ist;
wobei eine Positionierungsvorrichtung (1) nach einem der Ansprüche 1 bis 9 verwendet wird.

13. Verfahren nach Anspruch 12, **dadurch gekennzeichnet, dass** in der Sitzstellung der Körper der Personen zwischen Rumpf und Oberschenkel einen Winkel zwischen 80° und 100°, vorzugsweise zwischen 85°und 90°, aufweist.

## Claims

1. A positioning device (1) for positioning a physically handicapped person in a sitting position, preferably in a wheelchair, comprising
- a seat (3) having a base (5) and having a sitting surface (7) that can be displaced relative to the base (5),
- a dimensionally stable positioning collar (9), wherein the positioning collar (9) can be attached in a form-fit manner to the pelvic area of the person and contact surfaces (11) thereof rest on the iliac crest of the person in the attached state,
- traction means (15) connecting the positioning collar (9) to the base (5) of the seat (3), wherein the traction means (15) are each attached to one side area of the positioning collar (9) and comprise an actuating device (17),
- the traction means (15) exerting a tensile force in the direction of the sitting surface (7) and in a direction opposite the sitting direction when the positioning collar (9) is actuated, whereby the person can be drawn into the sitting position.

2. The positioning device of claim 1, **characterized in that** the positioning collar (9) is closed, wherein the positioning collar (9) preferably comprises a closure (13).

3. The positioning device of claim 1 or 2, **characterized in that** two traction means (15) are provided, wherein one traction means (15) is arranged on one side of the positioning collar (9), respectively.

4. The positioning device of one of claims 1 to 3, **characterized in that** the positioning collar (9) is of a multi-piece design, wherein the plurality of pieces of the positioning collar (9) can be connected or is connected by means of plug-in or hinge connections (19).

5. The positioning device of claim 4, **characterized in that** the positioning collar (9) consists of a plurality of tube parts.

6. The positioning device of one of claims 1 to 5, **characterized in that** the positioning device (9) comprises a backrest (21).

7. The positioning device of one of claims 1 to 6, **characterized in that** the traction means (15) are tensioning bands, tensioning belts, tensioning straps or drawbars.

8. The positioning device of one of claims 1 to 7, **characterized in that** the sitting surface (7) is adapted to be locked on the base (5), preferably in at least two positions.

9. The positioning device of one of claims 1 to 8, **characterized in that** the seat comprises a motor by which the sitting surface (7) can be driven to be adjusted, the motor preferably being an electric motor.

10. Use of a positioning device of one of claims 1 to 9 for the positioning of physically handicapped persons into a sitting position, preferably in a wheelchair (10).

11. The use as defined in claim 10, **characterized in that**, in the sitting position, the body of the person includes an angle between 80° and 100° between the torso and the upper legs, the angle preferably being between 85° and 90°.

12. A method of positioning physically handicapped persons in a sitting position, preferably in a wheelchair, **characterized by** the following steps:
- attaching a dimensionally stable positioning collar (9) in a form-fit manner in the pelvic area of the person such that contact surfaces (11) of the positioning collar (9) rest on the iliac crest of the person,
- sitting the person onto a slidable sitting surface (7),
- pulling the positioning collar (9) in the direction of the sitting surface (7) and in a direction opposite to the sitting direction and simultaneously sliding the sitting surface (7) in a direction opposite to the sitting direction, such that the person can be pulled to the sitting position;
wherein a positioning device (1) of one of claims 1 to 9 is used.

13. The method of claim 12, **characterized in that** in the sitting position, the body of the persons includes an angle between 80° and 100° between the torso and the upper legs, the angle preferably being between 85° and 90°.

## Revendications

1. Dispositif de positionnement (1) pour le positionnement d'une personne handicapée physique dans une position assise, de préférence dans un fauteuil roulant,
- avec un siège (3) avec une base (5) et avec une surface d'assise (7) pouvant coulisser par rapport à la base (5),
- avec une manchette de positionnement (9) indéformable, la manchette de positionnement (9) pouvant être mise en place par liaison de forme sur la zone de bassin de la personne et s'appuyant avec des surfaces d'appui (11) sur la crête iliaque de la personne dans l'état mis en place,
- avec des moyens de traction (15) qui raccordent la manchette de positionnement (9) à la base (5) du siège (3), les moyens de traction (15) étant fixés respectivement sur une zone latérale de la manchette de positionnement (9) et présentant un dispositif d'actionnement (17),
- les moyens de traction (15) exerçant sur la manchette de positionnement (9), lors de l'actionnement, une force de traction en direction de la surface du siège (7) et dans une direction opposée à la direction du siège, la personne pouvant être tirée dans la position assise.

2. Dispositif de positionnement selon la revendication 1, **caractérisé en ce que** la manchette de positionnement (9) est fermée, la manchette de positionnement (9) présentant de préférence une fermeture (13).

3. Dispositif de positionnement selon la revendication 1 ou 2, **caractérisé en ce que** deux moyens de traction (15) sont prévus, un moyen de traction (15) étant disposé respectivement sur un côté de la manchette de positionnement (9).

4. Dispositif de positionnement selon une des revendications 1 à 3, **caractérisé en ce que** la manchette de positionnement (9) est en plusieurs parties, les plusieurs parties de la manchette de positionnement (9) pouvant être raccordées ou étant raccordées les unes aux autres par des raccordements par enfichage ou articulés (19).

5. Dispositif de positionnement selon la revendication 4, **caractérisé en ce que** la manchette de positionnement (9) est composée de plusieurs parties tubulaires.

6. Dispositif de positionnement selon une des revendications 1 à 5, **caractérisé en ce que** le dispositif de positionnement (9) présente un appui dorsal (21).

7. Dispositif de positionnement selon une des revendications 1 à 6, **caractérisé en ce que** les moyens de traction (15) sont des bandes de serrage, des courroies de serrage, des sangles de serrage ou des barres de traction.

8. Dispositif de positionnement selon une des revendications 1 à 7, **caractérisé en ce que** la surface du siège (7) peut être arrêtée sur la base (5), de préférence dans au moins deux positions.

9. Dispositif de positionnement selon une des revendications 1 à 8, **caractérisé en ce que** le siège présente un moteur par le biais duquel la surface du siège (7) peut être entraînée pour le coulissement, le moteur étant de préférence un moteur électrique.

10. Utilisation d'un dispositif de positionnement selon une des revendications 1 à 9 pour le positionnement de personnes handicapées physique dans une position assise, de préférence dans un fauteuil roulant (10).

11. Utilisation selon la revendication 10, **caractérisée en ce que**, dans la position assise, le corps de la personne présente, entre le tronc et les cuisses, un angle compris entre 80° et 100°, de préférence entre 85° et 90°.

12. Procédé de positionnement de personnes handicapées physique dans une position assise, de préférence dans un fauteuil roulant, **caractérisé par** les étapes suivantes :
- mise en place par liaison de forme d'une manchette de positionnement (9) indéformable sur la zone de bassin de la personne de telle sorte que la manchette de positionnement (9) s'appuie avec des surfaces d'appui (11) sur la crête iliaque de la personne
- placement de la personne sur une surface du siège (7) pouvant coulisser
- traction de la manchette de positionnement (9) en direction de la surface du siège (7) et dans une direction opposée à la direction du siège et simultanément coulissement de la surface du siège (7) dans une direction opposée à la direction du siège de telle sorte que la personne peut être tirée dans la position assise ;
un dispositif de positionnement (1) selon une des revendication 1 à 9 étant utilisé.

13. Procédé selon la revendication 12, **caractérisé en ce que**, dans la position assise, le corps de la personne présente, entre le tronc et les cuisses, un angle compris entre 80° et 100°, de préférence entre 85° et 90°.
